(19) Europäisches Patentamt
European Patent Office
Office européen des brevets

(11) **EP 3 334 339 B1**

(12) **EUROPEAN PATENT SPECIFICATION**

(45) Date of publication and mention
of the grant of the patent:
**11.09.2019 Bulletin 2019/37**

(51) Int Cl.:
**A61B 5/083** (2006.01)    **A61B 5/097** (2006.01)
**G01N 33/497** (2006.01)    **A61M 16/04** (2006.01)

(21) Application number: **16751664.0**

(22) Date of filing: **27.07.2016**

(86) International application number:
**PCT/IB2016/054469**

(87) International publication number:
**WO 2017/025840 (16.02.2017 Gazette 2017/07)**

(54) **CAPNOGRAPHY WITH DECISION SUPPORT SYSTEM ARCHITECTURE**

KAPNOGRAFIE MIT ENTSCHEIDUNGSUNTERSTÜTZUNGSSYSTEMARCHITEKTUR

CAPNOGRAPHIE AVEC ARCHITECTURE DE SYSTÈME DE SUPPORT DE DÉCISION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.08.2015 US 201562203416 P**

(43) Date of publication of application:
**20.06.2018 Bulletin 2018/25**

(73) Proprietor: **Koninklijke Philips N.V.
5656 AE Eindhoven (NL)**

(72) Inventors:
• **ORR, Joseph
5656 AE Eindhoven (NL)**
• **BREWER, Lara Marie
5656 AE Eindhoven (NL)**
• **GUNNESON, Paul Bruce
5656 AE Eindhoven (NL)**

(74) Representative: **Müller, Frank
Philips Intellectual Property & Standards
High Tech Campus 5
5656 AE Eindhoven (NL)**

(56) References cited:
**US-A1- 2011 040 713    US-A1- 2013 204 099
US-A1- 2015 164 428**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The following relates generally to the capnography arts, medical monitoring arts, and related arts.

BACKGROUND

A capnography device monitors the concentration or partial pressure of carbon dioxide ($CO_2$) in respiratory gases. Capnography is commonly used in conjunction with mechanically ventilated patients in order to assess respiratory system status. A skilled anesthesiologist can evaluate the capnogram (that is, the $CO_2$ trend line as measured by a capnograph device) to assess respiratory health.

Capnography is increasingly used as a more generic vital sign for assessing patient health. For example, capnography may be used to monitor a patient who is breathing spontaneously and not undergoing mechanical ventilation, using a side stream capnograph device configuration in which respired air is sampled via a nasal cannula in conjunction with a dedicated sampling pump. In these broader contexts, medical personnel with limited expertise in anesthesiology are required to assess respiratory health on the basis of capnograph data. To facilitate this, it is common for the capnograph device to be programmed to output standard derived parameters, particularly respiration rate (RR) and end-tidal $CO_2$ (et$CO_2$). The RR is the breathing rate, quantified as the (quasi-)periodicity of the capnogram waveform. The et$CO_2$ is the partial pressure at the end of the exhalation phase. However, since the expired $CO_2$ is usually highest at the end of the exhalation phase, et$CO_2$ is commonly defined as the maximum observed $CO_2$ partial pressure over the breathing cycle.

While RR and et$CO_2$ are useful parameters, they do not capture the rich informational content of the capnogram waveform. To this end, it is also known to perform automated capnogram waveform analyses, designed to mimic clinical analyses that might be performed by a skilled anesthesiologist. For example, Colman et al., U.S. Pat. No. 8,412,655 and Colman et al., U.S. Pat. No. 8,414,488 disclose capnogram waveform analyses such as correlating pauses with apnea events, correlating a long downward slope of the capnogram waveform with possible partial airway obstruction, correlating a low capnogram waveform with possible low blood pressure, correlating a rounded capnogram waveform with a possible problem with the nasal cannula, or so forth. Based on such waveform analyses, the capnograph device may provide informational messages such as "open airway", "check airway", "check blood pressure", "check cannula interface", or so forth.

United States Patent Application Publication Number US 2015/164428A presents concepts for assessing quality for a variability analysis.

United States Patent Application Publication Number US 2011/0040713 A1 discloses a method of generating a pulmonary index value of a patient.

United States Patent Application Publication Number US 2013/0204099 A1 provides a medical monitoring device that includes one or more sensors.

Capnogram waveform analyses provide richer information from the capnogram, but entail complex processing such as detecting the breath cycling, amplitude and period normalization, and segmenting regions of the capnogram waveform within each breath cycle. These complex analyses introduce numerous possible error mechanisms such as incorrect waveform segmentation or information loss during the normalization operations.

The following discloses a new and improved systems and methods that address the above referenced issues, and others.

SUMMARY

According to an aspect of the invention, there is provided a capnograph device according to claim 1.

One advantage resides in providing a capnograph device whose output more effectively assesses patient respiratory health.

Another advantage resides in providing a capnograph device outputting derived parameters characterizing the detailed capnogram waveform without requiring breath detection or segmentation of the capnogram waveform.

Another advantage resides in more accurate respiratory system status information from capnogram data.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.

FIGURE 1 diagrammatically illustrates a capnograph device.
FIGURE 2 diagrammatically illustrates an idealized capnogram and the corresponding capnogram histogram.
FIGURE 3 diagrammatically plots the $CO_2$ fall time for an idealized capnogram waveform (top plot) and a capnogram with supplemental oxygen washout (bottom plot).

DETAILED DESCRIPTION

In some embodiments disclosed herein, parameter quality indices are computed to quantitatively assess the reliability of the respiratory rate (RR) and end-tidal $CO_2$ ($etCO_2$) evaluated from the capnogram. A respiratory well-being index (RWI) may also be computed, based in part on the $etCO_2$ parameter quality index ($etCO_2$ PQI) and the RR parameter quality index (RR PQI). These parameter quality indices enable medical personnel to interpret the capnogram using conventional tools, especially the RR and $etCO_2$, but provide metrics (the quality control indices) to assist medical personnel in assessing whether the RR and $etCO_2$ are reliable data for making clinical decisions.

Further, in some embodiments the parameter quality indices are computed at least in part using a histogram of $CO_2$ value counts versus (binned) $CO_2$ level. This histogram is computed over a time interval encompassing several breaths. For example, the histogram is acquired over a 30 second time interval in one illustrative embodiment, which corresponds to about 6-10 breaths for a normal adult patient respiration interval of 3-5 seconds/breath (12-20 breaths per minute), up to 30 breaths for a rapidly respiring infant (respiration rate of 60 breaths per minute).

Advantageously, the capnogram histogram is computed without segmenting the waveform into different regions (e.g. inspiration, expiration) and without segmenting individual breath cycles (that is, without a breath detector). The capnogram histogram advantageously has a "standard" shape for a normally respiring patient, due to the typical capnogram pattern in which the $CO_2$ level is close to zero during the inspiration phase and close to its maximum (i.e. close to $etCO_2$ for the patient) during the expiration phase. These two phases define respective low and high regions of the disclosed capnogram histogram, with a third transitional histogram region in-between. Rich information about the capnogram waveform can be extracted from the capnogram histogram, without reliance upon the difficult and often imprecise task of segmenting the capnogram waveform into breath cycles which are then further segmented into inspiration and expiration time intervals.

In particular, the $etCO_2$ PQI is computed primarily or entirely using the histogram. In some embodiments the $etCO_2$ PQI is computed further based on capnogram characteristics that can be quantified without segmenting the capnogram into inspiration and expiration regions. Illustrative embodiments of the RR PQI do rely upon breath detection and capnogram waveform segmentation, as the RR is intimately associated with (indeed defined by) the breath cycle. However, the RR PQI is optionally further based on the $etCO_2$ PQI thereby incorporating waveform information from the capnogram histogram.

The RWI is computed based on the $etCO_2$ and RR values, and further based on the $etCO_2$ PQI and RR PQI. Incorporating the PQI values into the RWI captures the recognition herein that a poor capnogram waveform is often an indication of poor respiratory health, rather than being an indicator of a capnograph measurement problem.

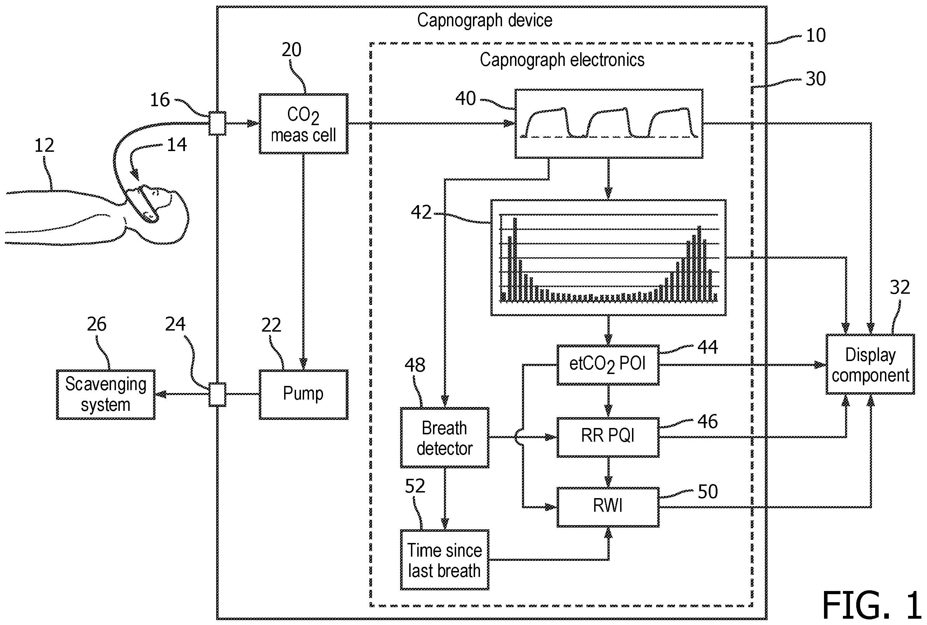

With reference to FIGURE 1, an illustrative capnograph device **10** is connected with a patient **12** by a suitable patient accessory, such as a nasal cannula **14** in the illustrative example, or by an airway adaptor or so forth. The patient accessory **14** may optionally include one or more ancillary components, such as an air filter, water trap, or the like (not shown). In the illustrative capnograph **10,** respired air is drawn from the patient accessory **14** into a capnograph air inlet **16** and through a carbon dioxide ($CO_2$) measurement component or cell **20** by an air pump **22**. The air is then discharged via an air outlet **24** of the capnograph **10** to atmosphere or, as in the illustrative embodiment, is discharged through the air outlet **24** into a scavenging system **26** to remove an inhaled anesthetic or other inhaled medicinal agent before discharge into the atmosphere. The $CO_2$ measurement component or cell **20** may, for example comprise an infrared optical absorption cell in which carbon dioxide in the respired air drawn from the patient accessory **14** produces absorption that is detected by an infrared light source/detector assembly.

The illustrative capnograph device **10** has a sidestream configuration in which respired air is drawn into the capnograph device **10** using the pump **22,** and the $CO_2$ measurement cell **20** is located inside the capnograph device **10**. That is, the sidestream capnograph device **10** includes, as a unit, the carbon dioxide measurement component **20,** the electronic processor **30,** and the pump **22** connected to draw respired air though the carbon dioxide measurement component **20**. The sidestream configuration is suitably used for a spontaneously breathing patient, i.e. a patient who is breathing on his or her own without assistance of a mechanical ventilator. In an alternative configuration, known as a mainstream configuration (not illustrated), the $CO_2$ measurement cell is located externally from the capnograph device housing, typically as a $CO_2$ measurement cell patient accessory that is inserted into the "mainstream" airway flow of the patient. Such a mainstream configuration may, for example, be employed in conjunction with a mechanically ventilated patient in which the $CO_2$ measurement cell patient accessory is designed to mate into an accessory receptacle of the ventilator unit, or is installed on an airway hose feeding into the ventilator. The disclosed approaches for quantitatively assessing parameter quality and patient respiratory well-being are readily applied either in conjunction with a sidestream capnograph device (as in the illustrative example of FIGURE 1) or in conjunction with a mainstream capnograph device.

With continuing reference to FIGURE 1, the capnograph device **10** (in either the illustrative sidestream configuration or in the alternative mainstream configuration) includes capnograph electronics **30** which provide power and control for operating the $CO_2$ measurement cell **20** and (in the sidestream configuration) the pump **22**. Note that the power and control links are not illustrated in diagrammatic FIGURE 1. The capnograph electronics **30** additionally perform processing of the $CO_2$ signal output by the $CO_2$ measurement cell **20,** as diagrammatically indicated in FIGURE 1 and as described herein. Clinical data output by the capnograph **10** are displayed on a display component **32,** stored in an electronic medical record (EMR) or the like, or otherwise utilized. The display component **32** may be a component of the capnograph or, as illustrated in FIGURE 1, the display component **32** may be an external display component connected to the capnograph **10**. For example, the external display component **32** may be a multi-function bedside patient monitor and/or a nurses' station patient monitor or so forth. It will be further appreciated that the capnograph may include numerous other components not illustrated in simplified diagrammatic FIGURE 1, such as a pressure gauge, flow meter, and so forth.

The capnograph electronics **30** may be variously implemented, such as by a suitably programmed electronic processor, e.g. a microprocessor or microcontroller of the capnograph **10**. While a single electronics unit **30** is illustrated, it is alternatively contemplated to employ various combinations of electronics, for example different electronic components may be operatively interconnected to implement a pump, power supply, infrared light source and detector, power supply (for the $CO_2$ measurement cell **20**), analog-to-digital conversion circuitry (to sample the infrared light detector of the $CO_2$ measurement cell **20**), and so forth. Still further, it is contemplated for the electronics that perform the capnograph data processing to be disposed outside of the capnograph device itself. For example, the capnograph data processing may be performed by electronics in another device (for example, the computer of a nurses' station that receives the $CO_2$ signal from the measurement cell **20,** or that receives a capnogram generated by the capnograph device and performs further processing). It will be still further appreciated that the capnograph data processing disclosed herein as being performed by the capnograph electronics **30** may be embodied by a non-transitory storage medium storing instructions that are readable and executable by the microprocessor, microcontroller, or other electronic processor to perform the disclosed capnograph data processing. Such non-transitory storage media may, by way of non-limiting illustration, include a hard disk drive or other magnetic storage medium, a flash memory, read-only memory (ROM) or other electronic storage medium, an optical disk or other optical storage medium, various combinations thereof, or so forth.

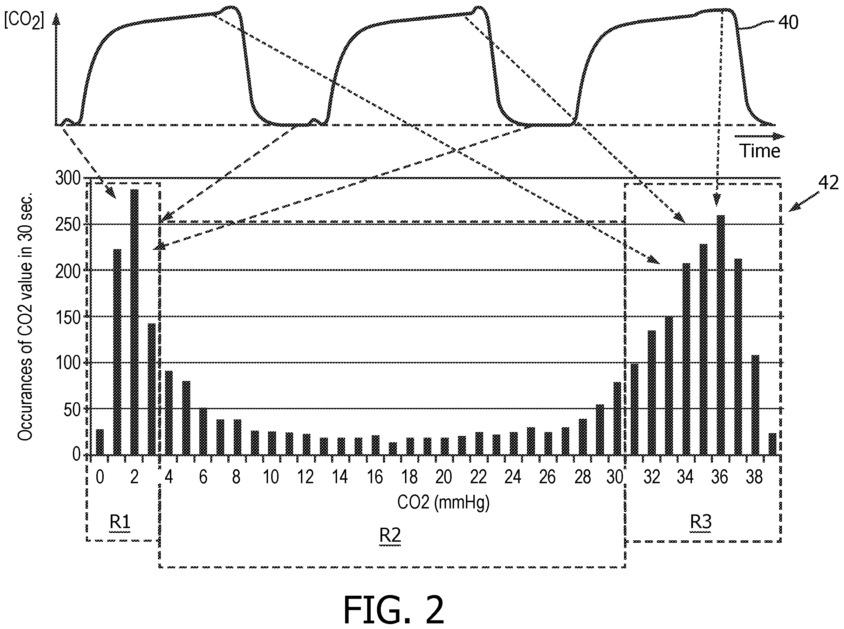

With continuing reference to FIGURE 1 and with further reference to FIGURE 2, an illustrative embodiment of the capnograph data processing performed by the capnograph electronics **30** (or alternatively in whole or in part by a nurses' station monitor, bedside patient monitor, or other device with a suitably programmed electronic data processor) is diagrammatically shown in FIGURE 1. The $CO_2$ signal is sampled and optionally corrected for factors such as the presence of interfering gases (e.g. nitrous oxide), barometric pressure, and so forth in order to generate a capnogram **40**. The capnogram **40** is a signal representing the partial pressure or concentration of carbon dioxide, denoted in FIGURE 2 as [$CO_2$], as a function of time. Diagrammatic FIGURE 2 illustrates the capnogram **40** as an idealized waveform for a healthy patient, in which every breath is identical and exhibits near-zero [$CO_2$] during the inspiratory phase and a well-defined maximum [$CO_2$] that rises gradually over the expiratory phase and terminates in a maximum [$CO_2$] corre-sponding to end-tidal $CO_2$, and in which the et$CO_2$ is the same for every breath. In practice, it will be understood that the capnogram **40** for a real patient usually deviates significantly from this idealized curve due to numerous factors such as non-uniform breathing, talking, coughing, possible chronic lung problems in the case of an ill patient, or so forth. In the capnogram of a real patient, the et$CO_2$ may vary from breath to breath. The illustrative idealized example of FIGURE 2 further assumes a constant respiration rate. Again, in a real patient, the RR is generally not constant - the RR can increase significantly due to excitement or exertion, may slow during rest periods, may stop entirely during a sleep apnea episode, and/or may generally vary significantly due to various respiratory ailments or other medical conditions.

With continuing reference to FIGURES 1 and 2, the capnograph electronics **30** are programmed to compute a capnogram histogram **42** from the capnogram **40**. The capnogram histogram **42** is a histogram of $CO_2$ sample values (y-axis) versus $CO_2$ level (x-axis). The capnogram histogram **42** is computed for a sliding window of duration 30 seconds (for illustrative FIGURE 2; other window sizes are contemplated, preferably of a duration long enough to encompass several breaths). By way of illustrative example, if the $CO_2$ measurement cell **20** acquires samples at 10 msec intervals (100 samples per second) and the window is 30 seconds then, for each capnogram sample in the 30 second window (consisting of 3000 points) the bin corresponding to the $CO_2$ value for the point is incremented. In the capnogram histogram of a typical capnogram, there is a lower baseline region during inspiration and an elevated $CO_2$ region during

expiration. Between these two regions, there are a set of points that make up the rising and falling edges of the capnogram. More particularly, as delineated in FIGURE 2, three regions **R1, R2, R3** can be defined. Region **R1** of the histogram **42** includes the points in the capnogram **40** measured by the $CO_2$ measurement cell **20** during the inspiratory phase of the breath. In the illustrative example of FIGURE 2, Region **R1** includes the bins from 0 to 3 mmHg. Region **R2** of the histogram **42** includes all the points from the capnogram **40** forming the rising and falling edges in the capnogram **40.** In the illustrative example of FIGURE 2, Region **R2** includes the bins from 4 to 30 mmHg. Finally, Region **R3** of the histogram **42** includes the points in the capnogram **40** measured during the expiratory phase of the breath. In the illustrative example of FIGURE 2, Region **R3** includes all the bins from 31 to 39 mmHg.

The capnogram histogram of a typical capnogram has certain characteristics. The histogram for a typical capnogram will have a higher number of occurrences of $CO_2$ sample values in the bins of Region **R1** and Region **R3,** and the number of occurrences in the bins of Region **R2** should be lower than the number of occurrences in Regions **R1** and **R3.** That is, the capnogram histogram **42** has a peak in lower Region **R1** and a peak in upper Region **R3,** and a valley in the intermediate Region **R2.** Further, the peak in upper Region **R3** is typically more spread-out than the peak in Region **R1,** as seen in the idealized capnogram histogram **42** of FIGURE 2. The spread in the peak in upper Region **R3** is caused by the slope of the capnogram **40** during the exhalation phase, with the highest $CO_2$ value typically occurring at the end of the breath (i.e. end-tidal point). This slope of the capnogram waveform **40** is reflected in spreading of the points making up the peak in upper Region **R3** of the capnogram histogram **42.** Such spreading may additionally or alternatively be caused by the usual situation in which every breath does not have the same peak $CO_2$ value (or, said another way, $etCO_2$ varies from breath to breath). The difference in $etCO_2$ value for each breath is reflected in spreading of the peak in upper Region **R3.** By contrast, during the inspiration phase of the capnogram the $CO_2$ level usually falls to a flat baseline level that is close to zero, and exhibits little variation from breath to breath, leading to a narrower peak in the lower Region **R1** of the histogram **42.**

The capnogram histogram **42** is computed from the capnogram **40** in the sliding window, with a new histogram computed every few seconds, e.g. every 5 seconds in one illustrative example employing a 30 second window. There is no attempt to synchronize the window with an integer number of breaths, but the window is preferably large enough to encompass several breaths (e.g. for a normal adult patient respiration interval of 3-5 seconds/breath the illustrative 30 sec window encompasses 6-10 breaths). By re-computing the histogram on a shorter time interval than the window size (e.g. every 5 sec using a 30 sec window) the successive histogram windows significantly overlap providing for a smoothing effect as a function of time. Since there is no synchronization with the breath cycling, there is no need to employ a breath detector in constructing the capnogram histogram **42,** and the determination of the histogram **42** is a very fast $CO_2$ sample binning process.

An end-tidal carbon dioxide ($etCO_2$) value and a respiratory rate (RR) value are determined from the capnogram signal **40.** Substantially any technique to detect a signal maximum can be used to detect the $etCO_2$ value. For example, in some embodiments, the $etCO_2$ value is determined from the capnogram signal **40** by analysis of the histogram **42** derived from the capnogram signal **40.** In this approach, the $CO_2$ level of the highest $CO_2$ level bin having a non-zero sample count provides an $etCO_2$ value. Similarly, substantially any technique to determine periodicity of a signal can be used to detect the RR value. For example, the RR value can be determined by detecting breaths using the breath detector **48** and thereby determining breath intervals (the RR being the inverse of the average breath interval). Alternatively, a Fast Fourier Transform (FFT) can be applied to determine the RR value in the frequency domain.

With continuing reference to FIGURE 1, the capnogram histogram **42** is used to compute an end-tidal $CO_2$ parameter quality index ($etCO_2$ PQI) **44.** This index is computed as a weighted sum of parameters derived from the capnogram histogram **42,** and optionally also from the capnogram **40** itself. The parameters included in the weighted sum are suitably chosen as relevant criteria in determining the confidence in the $etCO_2$ measurement obtained from the capnogram **40.** In one illustrative embodiment, the $etCO_2$ PQI **44** is computed from parameters including (1) a metric of the portion of the histogram **42** that is above the baseline; (2) a metric of the difference between the maximum $CO_2$ in Region **R3** and the $CO_2$ level in Region **R3** having the highest histogram counts; (3) a metric comparing the Region **R3** count versus the Region **R2** count; (4) a metric of the fraction of the total counts in Region **R3;** and (5) a metric of the $CO_2$ fall time.

The metric of the portion of the histogram **42** that is above the baseline characterizes the portion of the histogram that is in Region **R3** as compared with Region **R1.** This metric is large for a normal capnogram, but may be low in the case of a poor capnogram waveform having an inconsistent expiratory plateau.

The metric of the difference between the maximum $CO_2$ in Region **R3** and the $CO_2$ level in Region **R3** having the highest histogram counts is expected to be small because the end-tidal point should have the largest $CO_2$ value and a $CO_2$ level bin at or close to $etCO_2$ should also have a large number of counts since the expiratory plateau usually flattens as it approaches the end-tidal point. This metric may be computed from the difference between the $CO_2$ level of the bin of Region **R3** having a non-zero count and the $CO_2$ level of the bin of Region **R3** storing the highest count.

The metric comparing the upper Region **R3** count versus the intermediate Region **R2** count quantifies the expectation that a sharp transition should be present from the inspiratory phase to the expiratory phase in the capnogram **40.** In such a case, the intermediate Region **R2** count is low and the upper Region **R3** count is high. However, since there are more bins in intermediate Region **R2** than in upper Region **R3,** this metric may preferably be quantified using the average count over all bins of Region **R2,** and likewise using the average count over all bins of Region **R3.**

The metric of the fraction of the total counts in upper Region **R3** should be high since a large portion of the capnogram waveform consists of the expiratory phase. This metric may be computed using the ratio of the total counts in upper Region **R3** to the total counts in the capnogram histogram **42.**

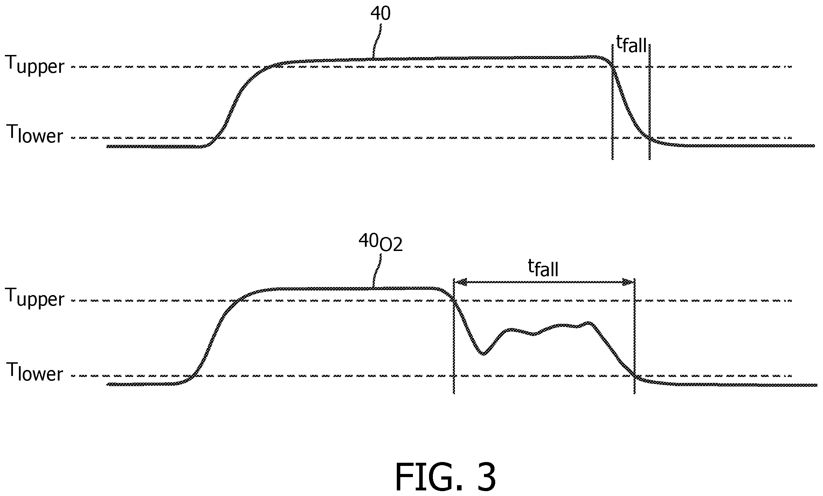

With brief reference to FIGURE 3, the metric of the $CO_2$ fall time differs from the previous four metrics contributing to the illustrative $etCO_2$ PQI in that the metric of the $CO_2$ fall time is computed from the capnogram **40** rather than from the capnogram histogram **42.** The metric of the $CO_2$ fall time is useful for detecting when the capnogram waveform is washed out due to the effect of supplemental oxygen. This is illustrated in FIGURE 3. The top plot of FIGURE 3 shows the expiratory plateau for the same idealized capnogram **40** as is shown in FIGURE 2. The $CO_2$ fall time is computed as the time interval from when a high $CO_2$ level falls below an upper threshold $T_{upper}$ until the $CO_2$ level decreases below a lower threshold $T_{lower}$. This $CO_2$ fall time is indicated as $t_{fall}$ in the top plot of FIGURE 3 showing the idealized capnogram **40.** It is seen that $t_{fall}$ is relatively short. By contrast, FIGURE 3 bottom plot shows a capnogram **40_{O2}** which exhibits supplemental oxygen washout. In this case, the transition from $T_{upper}$ to $T_{lower}$ is much longer.

It will be noted that the $CO_2$ fall time can be determined without performing breath detection, and without segmenting the capnogram waveform into inspiratory and expiratory phases. For example, in the illustrative example the $CO_2$ fall time is computed by identifying when a high $CO_2$ level falls below $T_{upper}$ and then when it falls below $T_{lower}$.

With returning reference to FIGURE 1, the $etCO_2$ PQI **44** is suitably computed as a weighted sum of these metrics (and/or other metric that correlate with the reliability of the $etCO_2$ measurement by capnography). That is:

$$etCO_2\ PQI = \sum_i W_i * S_i$$

where the index *i* ranges over the metrics contributing to $etCO_2$ PQI **44,** $S_i$ is the score (i.e. value) of the *ith* metric, and $W_i$ is a weight for the *ith* metric. The weights may be generated manually (e.g. based on assessment by a skilled pulmonologist of the relative importance of the various metrics) or by performing machine learning using a training set of representative capnograms each labeled by a skilled pulmonologist as to reliability of the $etCO_2$ value obtained from the training capnogram.

The five metrics contributing $etCO_2$ PQI in the example are merely illustrative. More generally, it will be appreciated that the capnogram histogram **42** is expected to exhibit a large narrow peak in a lower Region **R1** corresponding to the inspiration phase of the respiratory cycle, a large slightly broader peak in an upper Region **R3** corresponding to the expiratory phase, and a deep valley in an intermediate Region **R2** corresponding to transitions from inspiration-to-expiration and from expiration-to-inspiration. Deviations from this basic histogram shape are expected when the capnogram waveform is degraded, and consequently $etCO_2$ values are expected to be less reliable. Various metrics can be constructed and optimized using histograms constructed for training capnograms in order to quantitatively characterize metrics to assess the histogram shape and hence the capnogram waveform. The optimal choice of metrics, and their weights, depends on the capnograph device and its connection to the patient, the demographic being monitored, the desired sensitivity (e.g. how "bad" should the capnogram waveform be before the $etCO_2$ PQI starts to significantly decrease), and so forth. In some embodiments the metrics may be optimized for different patient connections (e.g. nasal cannula versus airway adaptor), different patient breathing conditions (e.g. spontaneous breathing versus various mechanical ventilation modes), or so forth. The capnogram histogram shape reflects the capnogram waveform, so quantitative metrics of the histogram provide assessment of the capnogram waveform quality without the need to detect breath intervals in the capnogram and without the need to segment the capnogram into inspiration and expiration phases. In the illustrative example, one metric ($CO_2$ fall time) is extracted directly from the capnogram **40** rather than from the capnogram histogram **42,** but this is still done without performing breath detection or segmenting the capnogram into breathing phases. The computations are fast, and can be performed in real-time (that is, with a delay of a few tens of seconds, a few seconds, or less).

With continuing reference to FIGURE 1, a respiratory rate parameter quality index (RR PQI) **46** is also determined. The RR, and the RR PQI, both depend on detecting breaths, and hence receive as input the breath intervals detected in the capnogram **40** by a breath detector **48.** The RR PQI **46** is suitably determined as a weighted sum of metrics including, by way of illustrative example: respiratory rate (RR), a metric of expiration time/inspiration time ratio (IE ratio), a metric quantifying invalid peak counts in the capnogram of a breath, a capnogram carbon dioxide level dynamic range metric, and a metric of how close the inspiratory $CO_2$ level is to zero. The RR and IE ratio values should be in reasonable ranges (e.g. RR around 12-20 breaths per minute for an adult) so values falling significantly outside the reasonable reduce the RR PQI **46.** Extra (invalid) peaks can result in erroneous breath detections, hence more invalid peaks reduces the RR PQI. The capnogram dynamic range (maximum $CO_2$ level minus minimum $CO_2$ level)

impacts the signal strength, so a low dynamic range reduces RR PQI. Similarly, the $CO_2$ level should be close to zero during inspiration; whereas, a higher $CO_2$ level during inspiration makes breath detection more difficult leading to a lower value for the RR PQI **46.**

In the illustrative embodiment of FIGURE 1, the RR PQI **46** is also determined based on the $etCO_2$ PQI **44** which serves as an additional metric in the weighted sum. The $etCO_2$ PQI **44** is a metric of "normalcy" of the capnogram waveform. Since a highly abnormal capnogram waveform makes breath detection more difficult, a lower value for the $etCO_2$ PQI **44** results in a lower RR PQI value as well. Employing the $etCO_2$ PQI **44** as an input metric to the RR PQI **46** advantageously re-uses the $etCO_2$ PQI **44** in assessing reliability of the RR.

The RR PQI **46** is again suitably computed as a weighted sum of the contributing metrics:

$$RR\ PQI = \sum_i W_i * S_i$$

where the index *i* ranges over the metrics contributing to RR PQI **46**, $S_i$ is the score (i.e. value) of the *ith* metric, and $W_i$ is a weight for the *ith* metric. The weights again may be generated manually or by performing machine learning using a training set of representative capnograms labeled as to RR reliability. The metrics contributing RR PQI in the example are again merely illustrative, and additional or other metrics are contemplated.

In some embodiments, a respiratory well-being index (RWI) **50** is also computed, which represents a quality score to assess the respiratory well-being of a patient using the capnogram **40**. The RWI **50** is designed to help medical personnel evaluate the overall respiratory well-being of the patient. RWI **50** may also be used to identify non-intubated patients who are at risk for hypoventilation due to central or obstructive apnea, such as during procedural sedation. In a suitable embodiment, metrics that serve as weighted inputs to the RWI **50** include the measured RR and $etCO_2$ and the corresponding RR PQI **44** and $etCO_2$ PQI **46**. In general, if either the RR or $etCO_2$ are outside their respective normal ranges then this lowers the RWI **50.** A lower RR PQI **44** or lower $etCO_2$ PQI **46** also lowers the RWI **50.** In some embodiments, a time-since-last-breath metric is also incorporated into the RWI **50** in order to facilitate its use in detecting airway obstruction or apnea episodes. For example, the time-since-last-breath may be quantified from the capnogram **40** by a block **52** assessing the time since last elevated $CO_2$ level.

The indices **44, 46, 50** are suitably re-calculated each time the capnogram histogram **42** is updated, e.g. every 5 seconds in the illustrative example. Since the illustrative histogram calculation window is 30 seconds, the first calculation of the indices **44, 46, 50** is performed after 30 seconds of the capnogram **40** are acquired.

If the capnograph device **10** is programmed to provide informational messages based on the RR and $etCO_2$ values, then the indices **44, 46, 50** may optionally be used to suppress these informational messages when the underlying RR or $etCO_2$ is unreliable as indicated by the corresponding PQI. By way of non-limiting illustration, in one contemplated embodiment the messaging scheme of Table 1 is employed, with the outputs only being displayed when RWI is lower than some threshold value.

**Table 1**

| Parameter | Message when the parameter is above the "Normal" range | Message when the parameter is below the "Normal" range |
|---|---|---|
| $etCO_2$ | "Hypo-ventilation" | "Shallow breaths" "try chin lift" |
| $etCO_2$ PQI Score | N/A | "Shallow breaths" "try chin lift" |
| RR | "Patient anxious" (do not show message if RR PQI is low) | "Low resp rate", "prompt patient to breathe" |
| RR PQI score | N/A | "Unstable breathing" |
| Time since high $CO_2$ signal | "Long time since breath", "prompt patient to breathe" | N/A |

In this illustrative messaging scheme, the "patient anxious" message is suppressed if the RR PQI **46** is below a threshold value.

In addition to (or in place of) computing and displaying (on the display component **32**) values of probative parameters such as $etCO_2$, RR, $etCO_2$ PQI **44,** RR PQI **46,** and/or RWI **50,** it is contemplated to display on the display component **32** the capnogram histogram **42** itself. As previously discussed, the capnogram histogram **42** embodies substantial information about the capnogram waveform in a format that may be more readily perceived by medical personnel as compared with reading a display of the capnogram **40** (which may optionally also be displayed on the display **32,** e.g. as a trend line). One advantage of displaying the capnogram histogram **42** as compared with displaying a trend line of the capnogram **40** is that the trend line is typically scrolled horizontally, whereas the capnogram histogram **42** does not scroll and is updated, e.g. every 5 seconds with substantial overlap between successive updates due to the large window overlap between successive updates (e.g. with a 30 second window and 5 sec updates, each successive histogram is derived from 25 seconds of the same capnogram data that was used to generate the immediately previous histogram and only 5 seconds of new capnogram data).

The invention has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

## Claims

1. A capnograph device (10) comprising:

   a carbon dioxide measurement component (20) configured to measure respiratory carbon dioxide level; and
   an electronic processor (30) programmed to:

   generate a capnogram signal (40) comprising carbon dioxide level sample values measured by the carbon dioxide measurement component as a function of time;
   determine an end-tidal carbon dioxide, $etCO_2$, value from the capnogram signal; and
   compute an end-tidal carbon dioxide parameter quality index, $etCO_2$ PQI, value (44) based on a waveform of the capnogram signal,
   **characterized in that** the electronic processor is configured to compute the $etCO_2$ PQI (44) by using at least one of:

   a metric of the $CO_2$ fall time from an upper carbon dioxide level threshold ($T_{upper}$) to a lower carbon dioxide level threshold ($T_{lower}$) and
   operations including generating a capnogram histogram (42) by binning counts of carbon dioxide level sample values of the capnogram signal (40) into carbon dioxide level bins.

2. The capnograph device (10) of claim 1, wherein the electronic processor (30) computes the $etCO_2$ PQI (44) using a metric of the $CO_2$ fall time from an upper carbon dioxide level threshold ($T_{upper}$) to a lower carbon dioxide level threshold ($T_{lower}$).

3. The capnograph device (10) of claim 1 or 2, wherein the electronic processor (30) is further programmed to:

   determine a respiration rate (RR) value from the capnogram signal (40); and
   compute a respiration rate parameter quality index, RR PQI, value (46) using the RR value and the $etCO_2$ PQI (44).

4. The capnograph device (10) of claim 3, wherein the electronic processor (30) is further programmed to compute the RR PQI (46) further using at least one of (i) a metric of expiration time/inspiration time ratio, (ii) a metric quantifying invalid peak counts in the capnogram of a breath, and (iii) a metric quantifying capnogram carbon dioxide level dynamic range.

5. The capnograph device (10) of claim 3 or 4, wherein the electronic processor (30) is further programmed to:
   determine a respiratory well-being index, RWI, (50) using the RR value, the $etCO_2$ value, the $etCO_2$ PQI (44), and the RR PQI (46).

6. The capnograph device (10) of claim 5, wherein the electronic processor (30) is programmed to compute the RWI (50) further using a time since last elevated $CO_2$ level determined (52) from the capnogram (40).

7. The capnograph device (10) of any one of claims 1-6, wherein the electronic processor (30) is programmed to compute the $etCO_2$ PQI (44) by operations including generating a capnogram histogram (42) by binning counts of carbon dioxide level sample values of the capnogram signal (40) into carbon dioxide level bins.

8. The capnograph device (10) of any one of claims 1-7, further comprising:
a display component (32) configured to display at least the determined $etCO_2$ value and the computed $etCO_2$ PQI value (44).

9. The capnograph device (10) of claim 1, wherein the electronic processor (30) is programmed to compute a quantitative capnogram waveform metric from the capnogram signal by operations including:

generating a capnogram histogram (42) from the capnogram signal wherein the capnogram histogram comprises carbon dioxide level bins storing corresponding counts of carbon dioxide level sample values in the capnogram signal; and
computing the quantitative capnogram waveform metric from the capnogram histogram;
determine the end-tidal carbon dioxide, $etCO_2$, value and a respiration rate, RR, value from the capnogram signal;
compute the end-tidal carbon dioxide parameter quality index, $etCO_2$ PQI, value (44) using the quantitative capnogram waveform metric; and
compute a respiration rate parameter quality index, RR PQI, value (46) using the RR value and the $etCO_2$ PQI (44).

10. The capnograph device (10) of claim 9, wherein the electronic processor (30) is programmed to determine the $etCO_2$ value from the capnogram signal (40) by operations including:
determining the $etCO_2$ value from the capnogram histogram (42) as the carbon dioxide level of the highest carbon dioxide level bin storing a non-zero count of carbon dioxide level sample values.

11. The capnograph device (10) of claim 9 or 10, wherein the quantitative capnogram waveform metric includes a metric of the portion of the capnogram histogram (42) that is above a baseline.

12. The capnograph device (10) of any one of claims 9-11, wherein the quantitative capnogram waveform metric includes a metric of the difference between the $CO_2$ level of a highest carbon dioxide level bin in an upper region (R3) of the capnogram histogram (42) storing a non-zero count and the $CO_2$ level of the carbon dioxide level bin in the upper region of the capnogram histogram storing the highest count of carbon dioxide level sample values.

13. The capnograph device (10) of any one of claims 9-12, wherein the quantitative capnogram waveform metric includes a metric comparing counts stored in all carbon dioxide level bins of an upper region (R3) of the capnogram histogram (42) and counts stored in all carbon dioxide level bins of an intermediate region (R2) of the capnogram histogram.

14. The capnograph device (10) of any one of claims 9-13, wherein the quantitative capnogram waveform metric includes a metric of the portion of all counts of the capnogram histogram (42) in an upper region (R3) of the capnogram histogram.

15. The capnograph device (10) of any one of claims 10-14, wherein the electronic processor (30) is programmed to compute the $etCO_2$ PQI (44) further using a metric of the $CO_2$ fall time from an upper carbon dioxide level threshold ($T_{upper}$) to a lower carbon dioxide level threshold ($T_{lower}$).

**Patentansprüche**

1. Kapnografvorrichtung (10), umfassend:

eine Kohlendioxid-Messkomponente (20), konfiguriert zum Messen des Atem-Kohlendioxidspiegels; und
einen elektronischen Prozessor (30), der programmiert ist zum:

Erzeugen eines Kapnogrammsignals (40), umfassend Kohlendioxidspiegel-Abtastwerte, die von der Kohlendioxid-Messkomponente in Abhängigkeit von der Zeit gemessen werden;
Bestimmen eines Atemzugende-Kohlendioxid-Wertes, $etCO_2$, aus dem Kapnogrammsignal; und
Berechnen eines Atemzugende-Kohlendioxid-Parameterqualitätsindex, etCO2-PQI, (44) basierend auf einer Wellenform des Kapnogrammsignals, **dadurch gekennzeichnet, dass** der elektronische Prozessor konfiguriert ist zum Berechnen von etCO2-PQI (44) unter Verwendung von mindestens einem der Folgenden: einer Metrik der $CO_2$-Abfallzeit von einem oberen Kohlendioxidspiegel-Schwellenwert ($T_{upper}$) auf einen unteren Kohlendioxidspiegel-Schwellenwert ($T_{lower}$) und Operationen, einschließlich des Erzeugens eines Kapnogramm-Histogramms (42) durch Klasseneinteilungszählungen von Kohlendioxidspiegel-Abtastwerten des Kapnogrammsignals (40) in Kohlendioxidklassen.

2. Kapnografvorrichtung (10) nach Anspruch 1, wobei der elektronische Prozessor (30) den etCO2-PQI (44) unter Verwendung einer Metrik der $CO_2$-Abfallzeit von einem oberen Kohlendioxidspiegel-Schwellenwert ($T_{upper}$) zu einem unteren Kohlendioxidspiegel-Schwellenwert ($T_{lower}$) berechnet.

3. Kapnografvorrichtung (10) nach Anspruch 1 oder 2, wobei der elektronische Prozessor (30) weiter programmiert ist zum:

   Bestimmen eines Wertes der Atemfrequenz (RR) aus dem Kapnogrammsignal (40); und
   Berechnen eines Wertes für den Atemfrequenz-Parameterqualitätsindex, RR-PQI, (46) unter Verwendung des RR-Wertes und des etCO2-PQI (44).

4. Kapnografvorrichtung (10) nach Anspruch 3, wobei der elektronische Prozessor (30) weiter programmiert ist zum Berechnen des RR-PQI (46) unter weiterer Verwendung von mindestens einem von (i) einer Metrik des Ausatmungszeit-Einatmungszeit-Verhältnisses, (ii) einer Metrik, die ungültige Spitzenwerte im Kapnogramm eines Atems quantifiziert, und (iii) einer Metrik, die den Kohlendioxidspiegel-Dynamikbereich des Kapnogramms quantifiziert.

5. Kapnografvorrichtung (10) nach Anspruch 3 oder 4, wobei der elektronische Prozessor (30) weiter programmiert ist zum:
   Bestimmen eines Atmungswohlfühlindex RWI (50) unter Verwendung des RR-Werts, des $etCO_2$-Werts, des etCO2-PQI (44) und des RR-PQI (46).

6. Kapnografvorrichtung (10) nach Anspruch 5, wobei der elektronische Prozessor (30) programmiert ist zum Berechnen von RWI (50) unter weiterer Verwendung einer Zeit seit dem letzten bestimmten erhöhten $CO_2$-Spiegel (52) aus dem Kapnogramm (40).

7. Kapnografvorrichtung (10) nach einem der Ansprüche 1-6, wobei der elektronische Prozessor (30) programmiert ist zum Berechnen von etCO2-PQI (44) durch Operationen, die das Erzeugen eines Kapnogrammhistogramms (42) durch Klasseneinteilungszählungen der Kohlendioxidspiegel-Abtastwerte des Kapnogrammsignals (40) in Kohlendioxidspiegelklassen einschließen.

8. Kapnografvorrichtung (10) nach einem der Ansprüche 1-7, weiter umfassend:
   eine Anzeigekomponente (32), die konfiguriert ist, um mindestens den bestimmten etCO2-Wert und den berechneten etCO2-PQI-Wert (44) anzuzeigen.

9. Kapnografvorrichtung (10) nach Anspruch 1, wobei der elektronische Prozessor (30) programmiert ist zum Berechnen einer quantitativen Kapnogramm-Wellenformmetrik aus dem Kapnogrammsignal durch Operationen, die Folgendes aufweisen:

   Erzeugen eines Kapnogramm-Histogramms (42) aus dem Kapnogrammsignal, wobei das Kapnogramm-Histogramm Kohlendioxidspiegelklassen umfasst, die entsprechende Zählungen von Kohlendioxidspiegel-Abtastwerten in dem Kapnogrammsignal speichern; und
   Berechnen der quantitativen Kapnogramm-Wellenformmetrik aus dem Kapnogramm-Histogramm;
   Bestimmen des Atemzugende-Kohlendioxidwerts, $etCO_2$ und eines Atemfrequenzwerts, RR, aus dem Kapnogrammsignal;
   Berechnen des Werts für den Atemzugende-Kohlendioxidparamater-Qualitätsindex, etCO2-PQI, (44) unter Verwendung der quantitativen Kapnogramm-Wellenformmetrik; und
   Berechnen eines Wertes für den Atemfrequenz-Parameterqualitätsindex, RR-PQI, (46) unter Verwendung des RR-Wertes und des etCO2-PQI (44).

10. Kapnografvorrichtung (10) nach Anspruch 9, wobei der elektronische Prozessor (30) programmiert ist zum Bestimmen des etCO2-Werts aus dem Kapnogrammsignal (40) durch Operationen, die Folgendes aufweisen:
    Bestimmen des $etCO_2$-Werts aus dem Kapnogramm-Histogramm (42) als Kohlendioxidspiegel der höchsten Kohlendioxidspiegelklasse, die eine Zählung der Kohlendioxidspiegel-Abtastwerten ungleich null speichert.

11. Kapnografvorrichtung (10) nach Anspruch 9 oder 10, wobei die quantitative Kapnogramm-Wellenformmetrik eine Metrik des Abschnitts des Kapnogramm-Histogramms (42) aufweist, der über einer Basislinie liegt.

12. Kapnografvorrichtung (10) nach einem der Ansprüche 9-11, wobei die quantitative Kapnogramm-Wellenformmetrik eine Metrik der Differenz zwischen dem $CO_2$-Spiegel einer Klasse mit dem höchsten Kohlendioxidspiegel in einem oberen Bereich (R3) des Kapnogramm-Histogramms (42), das eine Zählung ungleich null speichert, und dem $CO_2$-Spiegel der Kohlendioxidspiegelklasse in dem oberen Bereich des Kapnogramm-Histogramms, das die höchste Zählung von Kohlendioxidspiegel-Abtastwerten speichert, aufweist.

13. Kapnografvorrichtung (10) nach einem der Ansprüche 9-12, wobei die quantitative Kapnogramm-Wellenformmetrik eine Metrik aufweist, welche die in allen Kohlendioxidspiegelklassen eines oberen Bereichs (R3) des Kapnogramm-Histogramms (42) gespeicherten Zählungen vergleicht und die gespeicherten Zählungen in allen Kohlendioxidspiegelklassen eines Zwischenbereichs (R2) des Kapnogramm-Histogramms zählt.

14. Kapnografvorrichtung (10) nach einem der Ansprüche 9-13, wobei die quantitative Kapnogramm-Wellenformmetrik eine Metrik des Abschnitts aller Zählungen des Kapnogramm-Histogramms (42) in einem oberen Bereich (R3) des Kapnogramm-Histogramms aufweist.

15. Kapnografvorrichtung (10) nach einem der Ansprüche 10-14, wobei der elektronische Prozessor (30) programmiert ist zum Berechnen des etCO2-PQI (44) unter weiterer Verwendung einer Metrik der $CO_2$-Abfallzeit von einem oberen Kohlendioxidspiegel-Schwellenwert ($T_{upper}$) zu einem unteren Kohlendioxidspiegel-Schwellenwert ($T_{lower}$).

## Revendications

1. Dispositif de capnographe (10) comprenant :

   un composant de mesure de dioxyde de carbone (20) configuré pour mesurer un niveau de dioxyde de carbone respiratoire ; et
   un processeur électronique (30) programmé pour :

   générer un signal de capnogramme (40) comprenant des valeurs d'échantillonnage de niveau de dioxyde de carbone mesurées par le composant de mesure de dioxyde de carbone en fonction du temps ;
   déterminer une valeur de dioxyde de carbone en fin d'expiration, etCO$_2$, à partir du signal de capnogramme ; et
   calculer une valeur d'indice de qualité de paramètre de dioxyde de carbone en fin d'expiration, etCO$_2$ PQI (44), sur la base d'une forme d'onde du signal de capnogramme, **caractérisé en ce que** le processeur électronique est configuré pour calculer l'etCO2 PQI (44) en utilisant au moins une parmi :

   une métrique du temps de décroissance de CO$_2$ à partir d'un seuil de niveau de dioxyde de carbone supérieur ($T_{upper}$) jusqu'à un seuil de niveau de dioxyde de carbone inférieur ($T_{lower}$) et
   des opérations incluant la génération d'un histogramme de capnogramme (42) en collectant des décomptes de valeurs d'échantillonnage de niveau de dioxyde de carbone du signal de capnogramme (40) dans des cellules de niveau de dioxyde de carbone.

2. Dispositif de capnographe (10) selon la revendication 1, dans lequel le processeur électronique (30) calcule l'etCO2 PQI (44) en utilisant une métrique du temps de décroissance de CO$_2$ à partir d'un seuil de niveau de dioxyde de carbone supérieur ($T_{upper}$) jusqu'à un seuil de niveau de dioxyde de carbone inférieur ($T_{lower}$).

3. Dispositif de capnographe (10) selon la revendication 1 ou 2, dans lequel le processeur électronique (30) est en outre programmé pour :

   déterminer une valeur de fréquence respiratoire (RR) à partir du signal de capnogramme (40) ; et
   calculer une valeur d'indice de qualité de paramètre de fréquence respiratoire, RR PQI, (46) en utilisant la valeur RR et l'etCO2 PQI (44).

4. Dispositif de capnographe (10) selon la revendication 3, dans lequel le processeur électronique (30) est en outre programmé pour calculer le RR PQI (46) en utilisant en outre au moins une parmi (i) une métrique de rapport temps d'expiration/temps d'inspiration, (ii) une métrique quantifiant des pics de décompte invalides dans le capnogramme d'une respiration, et (iii) une métrique quantifiant une plage dynamique de niveau de dioxyde de carbone.

5. Dispositif de capnographe (10) selon la revendication 3 ou 4, dans lequel le processeur électronique (30) est en outre programmé pour :
déterminer un indice de bien-être respiratoire, RWI, (50) en utilisant la valeur RR, la valeur etCO$_2$, l'etCO2 PQI (44), et le RR PQI (46).

6. Dispositif de capnographe (10) selon la revendication 5, dans lequel le processeur électronique (30) est programmé pour calculer le RWI (50) en utilisant en outre un temps écoulée depuis le dernier niveau de CO$_2$ élevé déterminé (52) à partir du capnogramme (40).

7. Dispositif de capnographe (10) selon l'une quelconque des revendications 1 à 6, dans lequel le processeur électronique (30) est programmé pour calculer l'etCO$_2$ PQI (44) par des opérations incluant la génération d'un histogramme de capnogramme (42) en collectant des décomptes de valeurs d'échantillonnage de niveau de dioxyde de carbone du signal de capnogramme (40) dans des cellules de niveau de dioxyde de carbone.

8. Dispositif de capnographe (10) selon l'une quelconque des revendications 1 à 7, comprenant en outre :
un composant d'affichage (32) configuré pour afficher au moins la valeur etCO$_2$ déterminée et la valeur etCO$_2$ PQI calculée (44).

9. Dispositif de capnographe (10) selon la revendication 1, dans lequel le processeur électronique (30) est programmé pour calculer une métrique quantitative de forme d'onde de capnogramme à partir du signal de capnogramme par des opérations incluant les étapes consistant à :

générer un histogramme de capnogramme (42) à partir du signal de capnogramme dans lequel l'histogramme de capnogramme comprenant des cellules de niveau de dioxyde de carbone stockant des décomptes correspondants de valeurs d'échantillonnage de niveau de dioxyde de carbone dans le signal de capnogramme ; et calculer la métrique quantitative de forme d'onde de capnogramme à partir de l'histogramme du capnogramme ; déterminer la valeur de dioxyde de carbone de fin d'expiration, etCO$_2$, et une valeur de fréquence respiratoire, RR, à partir du signal de capnogramme ; calculer la valeur d'indice de qualité de paramètre de dioxyde de carbone en fin d'expiration, etCO$_2$ PQI,(44), en utilisant la métrique quantitative de forme d'onde de capnogramme ; et calculer une valeur d'indice de qualité de paramètre de fréquence respiratoire, RR PQI (46), en utilisant la valeur RR et l'etCO2 PQI (44).

10. Dispositif de capnographe (10) de la revendication 9, dans lequel le processeur électronique (30) est programmé pour déterminer la valeur etCO$_2$ à partir du signal de capnogramme (40) par des opérations incluant l'étape consistant à :
déterminer la valeur etCO$_2$ à partir de l'histogramme de capnogramme (42) en tant que niveau de dioxyde de carbone de la cellule de niveau de dioxyde de carbone la plus élevée stockant un décompte non nul de valeurs d'échantillonnage de niveau de dioxyde de carbone.

11. Dispositif de capnographe (10) selon la revendication 9 ou 10, dans lequel la métrique quantitative de forme d'onde de capnogramme inclut une métrique de la partie de l'histogramme de capnogramme (42) qui est au-dessus d'une ligne de base.

12. Dispositif de capnographe (10) selon l'une quelconque des revendications 9 à 11, dans lequel la métrique quantitative de forme d'onde de capnogramme inclut une métrique de la différence entre le niveau de CO$_2$ d'une cellule de niveau de dioxyde de carbone la plus élevée dans une région supérieure (R3) de l'histogramme de capnogramme (42) stockant un décompte non nul et le niveau de CO$_2$ de la cellule de niveau de dioxyde de carbone dans la région supérieure de l'histogramme de capnogramme stockant le décompte le plus élevé de valeurs d'échantillonnage de niveau de dioxyde de carbone.

13. Dispositif de capnographe (10) selon l'une quelconque des revendications 9 à 12, dans lequel la métrique quantitative de forme d'onde de capnogramme inclut une métrique comparant des décomptes stockés dans toutes les cellules de niveau de dioxyde de carbone d'une région supérieure (R3) de l'histogramme de capnogramme (42) et des décomptes stockés dans toutes les cellules de niveau de dioxyde de carbone d'une région intermédiaire (R2) de l'histogramme de capnogramme.

**14.** Dispositif de capnographe (10) selon l'une quelconque des revendications 9 à 13, dans lequel la métrique quantitative de forme d'onde de capnogramme comprend une métrique de la partie de tous les décomptes de l'histogramme de capnogramme (42) dans une région supérieure (R3) de l'histogramme de capnogramme.

**15.** Dispositif de capnographe (10) selon l'une quelconque des revendications 10 à 14, dans lequel le processeur électronique (30) est programmé pour calculer l'etCO$_2$ PQI (44) en utilisant en outre une métrique du temps de décroissance de CO$_2$ à partir d'un seuil de niveau de dioxyde de carbone supérieur (T$_{upper}$) jusqu'à un seuil de niveau de dioxyde de carbone inférieur (T$_{lower}$).

Capnograph device

Capnograph electronics

FIG. 1

EP 3 334 339 B1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8412655 B, Colman
- US 8414488 B, Colman
- US 2015164428 A
- US 20110040713 A1
- US 20130204099 A1